# EUROPEAN PATENT SPECIFICATION

(11) **EP 1 158 967 B1**
(45) Date of publication and mention of the grant of the patent: **16.04.2003**
(21) Application number: 00906508.7
(22) Date of filing: 29.02.2000
(51) Int. Cl.: A61K 9/70, A61K 47/10, A61K 47/14, A61K 31/196, A61K 31/405, A61K 31/5415, A61P 29/00

(54) **TRANSDERMAL DEVICE COMPRISING NON-STEROIDAL ANTI-INFLAMMATORY DRUGS INCORPORATED IN ACRYLIC ADHESIVE POLYMER MATRIX**
TRANSDERMALES SYSTEM ENTHALTEND NICHT-STEROIDISCHE ENTZÜNDUNGSHEMMER IN EINER ACRYLPOLYMER-KLEBEMATRIX
DISPOSITIF TRANSDERMIQUE COMPRENANT DES ANTI-INFLAMMATOIRES NON STEROIDIENS INTEGRES DANS UNE MATRICE POLYMERE ADHESIVE ACRYLIQUE

(30) Priority: 01.03.1999 AR 9910866
(43) Date of publication of application: 05.12.2001
(73) Proprietor: AMARIN TECHNOLOGIES S.A., 1232 Buenos Aires (AR)
(72) Inventor: STEFANO, Francisco, José, Evaristo, 1425 Buenos Aires (AR); SCASSO, Alejandro, Fabio, 1824 Provincia de Buenos Aires (AR); GABACH, Roberto, Juan, 1425 Buenos Aires (AR)
(74) Representative: Paget, Hugh Charles Edward
(86) International application number: GB0000707
(87) International publication number: WO00051575

(56) References cited:
- WO-A-94/15609
- WO-A-94/23713
- WO-A-99/51212
- GB-A- 2 273 044
- US-A- 4 390 520

## Description

### Technical Field

This invention pertains to transdermal administration devices, and more particularly, to transdermal administration devices for the systemic administration of a NSAID which employ a percutaneous permeation enhancer and a pressure sensitive acrylic adhesive matrix in which the NSAID is incorporated.

### Background of the Invention

Physicians use a variety of pharmaceutical compositions that contain non-steroidal anti-inflammatory drugs (NSAIDs) for the treatment of inflammatory and/or painful diseases, especially musculoskeletal processes. Within those compositions, the most commonly used are the dosages forms for the oral administration (tablets, film coated tablets, capsules, etc.), being less commonly used the injectable or rectal dosage forms. In all of these dosages forms or medicines, after the dissolution of the active drug, its passage to the circulating blood assures its systemic distribution through the whole body. This systemic or general distribution enables the active drug to arrive to the desired site (ill or damaged tissues) as well as distributes it to the rest of the body. The arrival of the active drug to the damaged tissue allows it to perform its therapeutic action, while its presence in the rest of the body may be the reason of adverse or toxic effects. Anti-inflammatory and analgesic agents with a non-steroidal structure produce several types of undesirable effects, being the aggression to the mucosa of the gastrointestinal track the most common, with its consequent irritative effects, ulcerations and haemorrhages. [1] For most of these events the frequency is higher, and a fatal outcome is more probable, in elderly patients than in younger patients. [2]

To avoid or reduce the systemic adverse effects, mainly the gastrointestinal damage, it has became popular among physicians and patients to use or administration topical compositions (gels, ointments, creams) with variable concentrations of anti-inflammatory drugs.[3] Such compositions are the subject of many patents, for example, US 5,472,982, US 5,350,769, US 5,824,658, US 5,527,832, US 5,164,416, US 4,670,254, US 4,917,886, US 5,422,102, US 5,374,661, US 4,545,992, US 5,795,916 and JP 9208463.

In patent EP 428352, the applicant discloses the use of fatty acids (C₄-C₃₀) and alkyl and alkenyl esters thereof (C₄-C₃₀) to enhance the percutaneous penetration of non-steroidal anti-inflammatories in creams, ointments or gels.

In addition to these semi-solid dosage forms, there are other patents that disclose solid presentations or plasters.

Patent US 5,607,690 describes the manufacture of a plaster comprising a salt of diclofenac with a cyclic organic base, and their local anti-inflammatory and analgesic effects are also claimed.

Patent US 4,390,520 claims a flexible polymer substrate that comprises indomethacin, to release the active drug through the skin with the aim of treating affected tissues in the vicinity of the application site.

Also, patent EP 524582 B1 discloses the composition of a diclofenac sodium plaster that comprises a penetration enhancer composed of 1-menthol and propylene glycol, for which the authors claim a good percutaneous absorption of the active drug.

Patent US 4,738,848 discloses an adhesive preparation comprising a flexible support which contains a combination of diclofenac sodium and an organic acid that facilitates the solubilisation of the active drug and its percutaneous absorption.

However, the previous patents appear to be restricted to effects nearby the site of action and do not mention any general or systemic therapeutic effect, nor do they present experiments in human related to such mentioned systemic action of the topical applied active drug. Thus, the uses described in the aforementioned patents would be limited to the treatment of local diseases in the vicinity of the site of application.

In spite of the fact that in many patents the transdermal administration of anti-inflammatories is mentioned (EP 379,045 B1, US 5,662,925, US 5,674,521, US 5,336,213, US 5,132,115), there are no references or specific teachings to evaluate the efficacy of the disclosed products (devices) or the methodologies relating to the manufacture of those products (devices).

Patent US 4,999,379 discloses the systemic administration of diclofenac through a transdermic composition that specifically contains two permeation enhancers, N, N-dimethyllauroylamide or 1-N-dodecylazacycloheptan-2-one. In its examples related to matrix patches, the active drug is micro-suspended, which means that its absorption from the matrix could be erratic, since it is not solubilised. On the other hand, it does not claim the systemic effect achieved by the release of low and steady concentrations of the active drug. Also, it does not indicate the use of acrylic adhesive or the permeation data of the drug released from the transdermic device. Moreover, no indication of the plasmatic levels reached or the therapeutic effects are informed.

Patent US 5,665,378 is related to the transdermal administration of several NSAIDs (including diclofenac sodium), capsaicin and pamabrom, using menthol, eucalyptol, glyceryl monostearate and d-limonene as permeation enhancers. This patent discloses a reservoir type transdermal system and does not claim the presence of steady plasmatic concentrations to yield systemic effects.

Patent EP 827741 A2 discloses a composition for the transdermal release of a NSAID such as propionic acid derivatives (ketoprofen, etc.), wherein the drug is dispersed in non-polar adhesive polymers, in which an increase of the skin permeability is found because of the different physico-chemical characteristics that exist between these drugs and the mentioned adhesives. This patent does not have information related to the systemic effect of the device, plasmatic concentrations or experiments of therapeutic efficacy.

The anti-inflammatory reaction is recognised as a defence mechanism of the body against external or internal aggressions. It is initiated by the local secretion and release of several compounds synthetised by the cells involved in the defence reaction as interleukines, bradykinin, and prostaglandins. The pharmacological action of currently available NSAIDs is related to inhibition of the enzyme cyclooxygenase, which transforms a fatty acid present in the cell membranes (arachidonic acid) to intermediates that will result in different types of prostaglandins that initiate and maintain the anti-inflammatory response. [1]

The inhibition of this enzyme could be irreversible, as the one produced by acetylsalicylic acid or competitive as the one produced by ibuprofen. In the first case the exposure of the enzyme at low concentrations of the inhibitor for a long time, will yield a degree of inhibition similar to the one obtained when the enzyme is exposed to high concentrations of the inhibitor for short periods of time. In contrast, for the competitive inhibitors, the amount of inhibition is a function of the concentration of the anti-inflammatory and it is independent of the time of exposure.

### Summary of the Invention

We have found that for the non-steroidal anti-inflammatory drug diclofenac the enzymatic inhibition of the cyclooxygenase, measured as the anti-inflammatory response, can be achieved if the pharmaceutical composition releases low and steady plasmatic concentrations of NSAID (constantly) and for prolonged periods of time. The described release is obtained if the NSAID is dissolved in an autoadhesive matrix of an acrylic copolymer comprising -COOH groups and adding to the compositions a compound that control the percutaneous permeation. Consequently, in this manner, the obtained pharmaceutical composition has simultaneously the therapeutics advantages of the oral administration of the NSAID and the low incidence of side effect attributed to the local dosage forms. Additionally, this invention has the well-known advantages of transdermal systems.

Many strategies have been suggested to overcome the low skin permeability of NSAIDs and it is very well known that the selection of suitable vehicles is an important factor in the percutaneous absorption of the drug.

In our invention, the combination of the NSAID with already known compounds that modify the skin permeation, specifically fatty alcohols (C₄-C₃₀), mono, di or triglyceryl fatty acids esters (C₄-C₃₀) , in a matrix type transdermal device (in which the matrix is an acrylic pressure sensitive adhesive copolymer with polar functional groups), yields a suitable permeation of the active drug.

The novelty of our invention consists in the achievement of lower and steadier plasmatic concentrations than those obtained with other systemic pharmaceutical dosages, being these concentrations able to yield similar therapeutic efficacy than the other dosage forms with a decrease of undesirables effects. So, it is not our objective to obtain high permeation fluxes of the drug to yield therapeutic efficacy, because we have unexpectedly found that it is possible to perform a therapeutic effect by the maintenance of low and steady plasmatic concentration of the active drug trough the whole period of administration.

It is, therefore, an object of this patent to describe compositions of a non-steroidal anti-inflammatory drug in combination with modifiers of the percutaneous permeation in transdermal systems in which the pressure sensitive polymeric adhesive matrix has polar functional groups, that have the property of producing steady plasmatic concentrations of the active drug capable to obtain a systemic anti-inflammatory and analgesic effect and avoiding the limitation of its use only in the vicinity of the affected structure (joint or muscle).

Summing up, in the present invention we describe compositions comprising diclofenac in combination with modifiers of the percutaneous permeation that are incorporated into pressure sensitive adhesive polymeric matrix with polar functional groups, which have the property of releasing steady plasmatic concentrations of the active drug capable to obtain a systemic effect and avoiding the limitation of its use only in the vicinity of the affected structure (joint or muscle).

### Detailed Description of the Invention

In the transdermal device described in the present invention, the NSAID diclofenac or a pharmaceutically acceptable salt thereof, is the active drug, which is dissolved and incorporated homogeneously into the adhesive polymeric matrix. It is especially important to take into account the compatibilty of the active drug with the polymeric matrix in the process of preparation of the mixture in order to guarantee the physical and chemical stability of the product.

The preferred concentration of the active drug is between the 4.5 and 32% by weight of the polymeric matrix.

Also, modifiers of the percutaneous permeation which facilitate the dissolution or modify the properties of the stratum corneum modulating the transfer of the NSAID to the circulation, are added. These type of substances are selected from fatty alcohols (C₄-C₃₀), and mono-di or triglyceryl fatty acid esters (C₄-C₃₀) preferably with a concentration not higher than 35%, because an excess of these compounds decreases the adhesive properties and may produce irritation of the skin.

As for pressure sensitive adhesives, we have selected those with -COOH functional groups. Particularly preferred are those polymers with a predominance of carboxylic acid groups and minimal presence of hydroxyl groups and there is little or no need of addition of cross-linking agents (as organic salts of transition metals). Examples of such adhesives include polyacrylate adhesives, which are produced by the copolymerization of acrylic acid, acrylic esters, and other functional monomers.

The preferred adhesives used for the invention are sold by National Starch & Chemical Company, but the use of adhesives from other companies with similar characteristics is also possible as would be known by anyone skilled in the art. Examples of suitable adhesives include the following, from National Starch & Chemical Company:
DT 87-2852, which contains:
   2-ethylhexylacrylate: 65% (primary monomer) ;
   methyacylate: 28% (modifying monomer);
   acrylic acid: 7% (monomer with functional group);
   aluminum acetylacetonate (crosslinker);
   functional group: -COOH.
DT 87-2353, which contains:
   2-ethylhexylacrylate: 62% (primary monomer) ;
   methyacylate: 32% (modifying monomer);
   acrylic acid: 6% (monomer with functional group) ;
   glycidylmethacrylate: <1%;
   functional group: -COOH.
DT 87-2070, which contains:
   2-ethylhexylacrylate: 64% (primary monomer) ;
   vinyl acetate: 35% (modifying monomer);
   acrylic acid: 1% (monomer with functional group) ;
   2-hydroxyethylacrylate: <1% (monomer with functional group);
   aluminum acetylacetonate (crosslinker);
   functional group: -COOH/-OH.

Because of the general characteristics of these types of combinations, the protection against oxidation using antioxidants and stabilising agent as butylhydroxytoluene, butylhydroxyanisol, ascorbic acid, tocopherols, lecitin, polyvinyl pyrrolidone, gum guar, or carboxymethylcellulose may be necessary.

Sometimes, the addition of fillers, such as bentonite, titanium dioxide, talc, or silicon dioxide has been useful, but the concentration of filler is preferably lower than 7.5%.

The transdermal system, typical of this invention, is obtained when a homogeneous mixture is obtained from the combination of the aforementioned compounds. The obtained mixture is coated (with a thickness preferably no greater than 425 µm) onto a polyester, cellulose acetate, polyvinyl chloride, polyethylene, metalled polyethylene liner or siliconized or fluoropolymerized or properly treated vinyl-ethylene acetate copolymer in order to have a non-adhesive phase.

The coated film is then dried in a continuous processing oven. The conditions in the drying process are adequate to produce an homogeneous evaporation of the solvents avoiding the presence of solvent trapped into the polymeric matrix, which can cause bubbles, craters or folds that can alter the aesthetic appearance. The residence time in the oven is optimised so as not to alter or degrade any of the components or the structure of the liner but assure the complete curing of the used adhesive. Typically, the conditions to fulfil the former objectives are temperatures lesser to 110°C and residence times less than 12 minutes.

After its exit from the oven, the structure that contains the dry adhesive is laminated with the backing liner. Suitable materials for the backing liner include polyethylene, polyester, vinyl-ethylene acetate copolymer, polyurethane, polyvinylalcohol copolymer, non woven fabrics, or multilayer films of the mentioned materials.

The multilayered laminate is conveyed into rolls of suitable size and taken to a die-cutting machine, which may be rotary or any other suitable type. By using suitable cutting tools it is possible to obtain transdermal devices of a variety of shapes and active surfaces in order to assure effective dosages of the active drug. The obtained transdermal devices are conditioned in their final packaging.

The devices that are the objects of this invention a preferably applied on non-irritated skin areas and free of hair (i.e. shoulders, forearms, arms, chest, abdomen, gluteal, etc.). Preferably, they are not applied in areas of the body where the stratum corneum is thick (i.e. palm of hand, sole of the foot).

Examples 1 to 8 describe compositions based on this invention, in which the composition of the formula is expressed as percent of the weight of the total content of the dry coating, i.e. excluding adhesive solvents.

Examples 9 to 13 show experimental results that illustrate permeation, plasmatic concentrations and systemic effect of the compositions in humans.

Examples 10 to 12 demonstrate that the invention achieves an evident improvement of the evaluated parameters, essentially pain, in which it is similar to the reported for the oral dosages of several NSAIDs.

In Example 13, it is observed that the plasmatic concentrations of the active drug achieved with our invention are steady over the application period of 24 hours and they are much lower than the ones achieved with oral or injectable preparations in accordance with the pharmacokinetics data found in bibliography.

All this sustains the novelty of our invention, in which an efficacy similar to other systemic dosage form is achieved, but with low and steady levels of the active drug in the blood.

### Example 1

A mixture of 79.45 parts of adhesive, 10 parts of NSAID, 10 parts of permeation modifier and 0.55 parts of antioxidants are slowly mixed with stirring to minimise the incorporation of air. It is feasible that an increase of the temperature of the mixture be necessary to dissolve the components. The obtained mixture is coated onto the release liner, dried and then covered with the backing layer. The resulting composition has the following compounds in the indicated quantity:

| **Compound** | **%** |
|---|---|
| Adhesive DT 87-2852 | 60 |
| Diclofenacdiethylammonium (DDA) | 15 |
| Oleyl alcohol (OA) | 25 |

In the following examples the method of example 1 is used with the appropriate starting material to obtain compositions with the following compounds:

### Example 2

| **Compound** | **%** |
|---|---|
| Adhesive DT 87-2852 | 64.45 |
| DDA | 25 |
| OA | 10 |
| BHT (Butylhydroxytoluene) | 0.5 |
| BHA (Butylhydroxyanisol) | 0.05 |

### Example 3

| **Compound** | **%** |
|---|---|
| Adhesive DT 87-2852 | 64.45 |
| DDA | 25 |
| Glyceryl monooleate (GMO) | 10 |
| BHT | 0.5 |
| BHA | 0.05 |

### Example 4

| **Compound** | **%** |
|---|---|
| Adhesive DT 87-2353 | 64.45 |
| DDA | 25 |
| GMO | 10 |
| BHT | 0.5 |
| BHA | 0.05 |

### Example 5

| **Compound** | **%** |
|---|---|
| Adhesive DT 87-2852 | 63.45 |
| DDA | 25 |
| GMO | 10 |
| BHT | 0.5 |
| BHA | 0.05 |
| Polyvinylpyrrolidone (PVP K-30) | 1 |

### Example 6

| **Compound** | **%** |
|---|---|
| Adhesive DT 87-2852 | 59.45 |
| DDA | 25 |
| GMO | 10 |
| BHT | 0.5 |
| BHA | 0.05 |
| Bentonite NF | 5 |

### Example 7

| **Compound** | **%** |
|---|---|
| Adhesive DT 87-2353 | 59.45 |
| DDA | 25 |
| GMO | 10 |
| BHT | 0.5 |
| BHA | 0.05 |
| Bentonite NF | 5 |

### Example 8

| **Compound** | **%** |
|---|---|
| Adhesive DT 87-2070 | 69.45 |
| DDA | 15 |
| GMO | 15 |
| BHT | 0.5 |
| BHA | 0.05 |

### Example 9

Permeation experiments through mouse skin were performed using the transdermal device obtained as in Example 2 and a commercial plaster of local action (Dioxaflexâ, lot 960212, made in Switzerland), which results are in Table 1:

**TABLE 1**

| **Time (hours)** | **Amount of permeated diclofenac (cumulative) (µg/cm**^{**2**}**)** | |
|---|---|---|
| | **Dioxaflex** | **Transdermal device according to Example 2** |
| 4 | 1.29 ± 0.38 | 4.82 ± 0.53 |
| 8 | 3.28 ± 0.93 | 24.29 ± 4.94 |
| 24 | 13.02 ± 4.91 | 80.36 ± 6.52 |

This comparative experiment shows the higher permeation of the product elaborated as in Example 2.

### Example 10

The therapeutic efficacy of the transdermal devices elaborated as in Example 1 was applied in 9 patients suffering knee osteoarcrosis. The trial had an open design and the patients were required to indicate the degree of pain on a graphic scale (Visual Analogue Scale method [4]). The results are shown in Table 2.

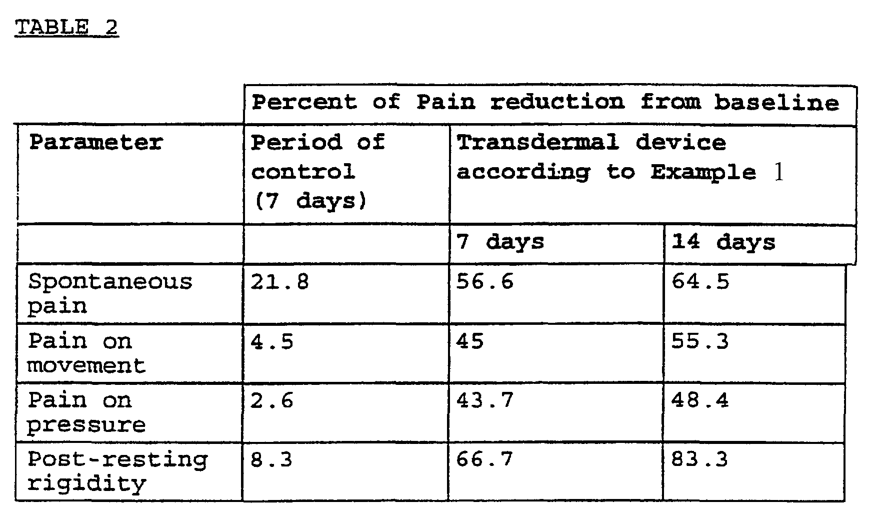

During the period of control the patients were only medicated with oral paracetamol. The applied devices contained 100 mg of DDA in a surface of 100 cm² and were replaced each 24 hours.

### Example 11

Experiment with a design similar to the one of Example 10 using a device elaborated as in Example 2. The participating patients (n = 7) had the same pathology. The results are shown in Table 3.

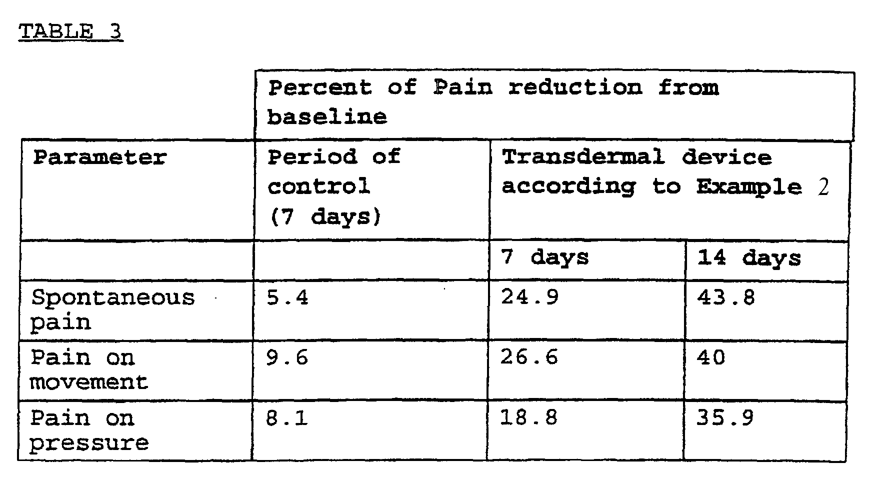

The applied devices contained 100 mg of DDA in a surface of 100 cm² and were replaced every 24 hours.

### Example 12

Experiment with a design similar to Example 11 using a device elaborated as in Example 2, performed over 33 patients with the same pathology. The results are shown in Table 4.

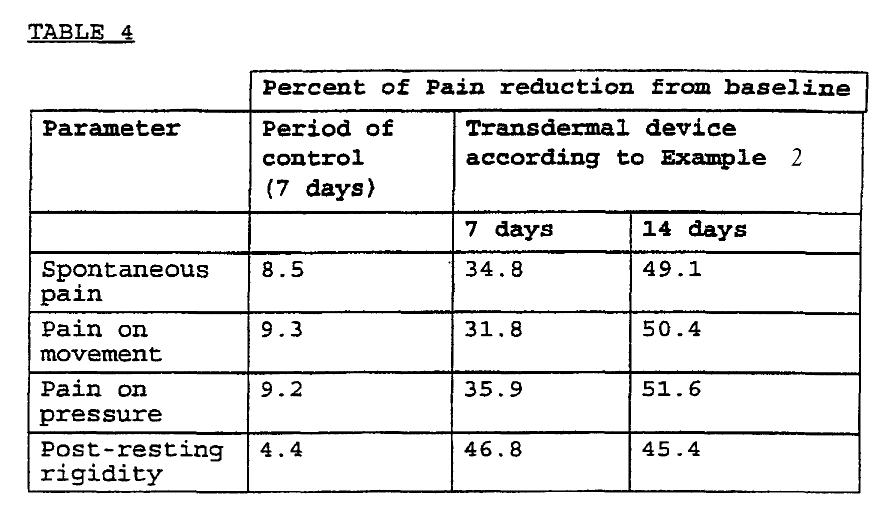

In this case, the applied devices contained 100 mg of DDA in a surface of 50 cm². The transdermal devices were replaced each 24 hours.

### Example 13

The plasmatic levels of diclofenac obtained after the second application of a transdermal device similar to the one used in Example 12 were determined in 14 healthy volunteers. The samples were measured by GC-MS and the results are shown in Table 5.

**TABLE 5**

| | | | |
|---|---|---|---|
| **Time after the second application** **(hours)** | 4 | 12 | 24 |
| **Plasmatic concentration of diclofenac** **(nmol/liter)** | 47.8 (7.6)* | 40.0 (5.1)* | 34.7 (4.8)* |

| | | | |
|---|---|---|---|
| *Standard Error of the Mean (SEM) | | | |

These results show that the transdermal device brings low (compared with the oral or injectable dosage forms), but steady plasmatic concentrations of the active drug.

### References

[1] Paul A. Insel in "Goodman & Gilman's, The Pharmacological basis of therapeutics", ch. 27 (Analgesic and Anti-inflammatory agents and drugs employed in the treatment of gout), pp. 617-658, 9^{th} edition, Mc-Graw-Hill, USA, 1996.
[23 Evans, J.M.M.; MacDonald, T. M., Tolerability of topical NSAIDs in the elderly, Drugs Aging (1996) 9 (2), pp. 101-108
[3] Halpern, S M, Topical non-steroidal anti-inflammatory drugs: a review of their use and toxicity, J. Dermatol. Treat (1994), 5, pp. 103-7.
[4] M. Lequesne, Methodology Issues in the Evaluation of NSAIDS in Inflammatory Rheumatic Diseases, Journal of Rheumatology (1990); (Supplement 20), vol. 17 pp.25-28.

## Claims

1. A transdermal administration device that contains:
(a) a pharmaceutical composition for the systemic administration of a NSAID which is diclofenac or a pharmaceutically acceptable salt thereof;
(b) a backing layer; and,
(c) a release liner;
wherein said pharmaceutical composition includes:
at least one component that enhances percutaneous permeation selected from:
(i) fatty alcohols (C₄-C₃₀); and
(ii) mono, di or triglyceryl fatty acid esters (C₄-C₃₀); and
a pressure sensitive acrylic adhesive matrix formed by a copolymer containing -COOH groups, in which the NSAID is incorporated.

2. A transdermal administration device according to claim 1, wherein the NSAID is present in an amount in the range 4.5 to 32% by weight of total content excluding adhesive solvents.

3. A transdermal administration device according to claim 2, wherein the NSAID is the diclofenac diethylammonium salt.

4. A transdermal administration device according to any one of claims 1 to 3, wherein the pressure sensitive adhesive matrix contains methacrylic acid or an ester thereof, at least one other copolymerisable monomer and cross-linking agent, and said pressure sensitive adhesive polymeric matrix is present in an amount in the range 50 to 97.5% by weight of total content excluding adhesive solvents.

5. A transdermal administration device according to claim 4, wherein the pressure sensitive acrylic mixture is a copolymer of 2-ethyl-hexyl-acrylate, methyl-acrylate, acrylic acid, and glycidylmethacrylate.

6. A transdermal administration device according to any one of claims 1 to 5, wherein the permeation enhancer is oleyl alcohol, and is present in an amount in the range 5.5 to 35% by weight of total content excluding adhesive solvents.

7. A transdermal administration device according to any one of claims 1 to 5, wherein the permeation enhancer is glyceryl monooleate, and is present in an amount in the range 5.5 to 35% by weight of total content excluding adhesive solvents.

8. A transdermal administration device according to any one of claims 1 to 7, wherein said copolymer contains -OH groups.

## Patentansprüche

1. Vorrichtung zur transdermalen Verabreichung, die Folgendes umfasst:
(a) eine pharmazeutische Zusammensetzung zur systemischen Verabreichung eines NSAID, das Diclofenac oder ein pharmazeutisch annehmbares Salz davon ist;
(b) eine Stützlage; und
(c) eine Abziehlage;
worin die pharmazeutische Zusammensetzung Folgendes umfasst:
zumindest eine Komponente, die die perkutane Penetration verstärkt, ausgewählt aus:
(i) Fettalkoholen (C₄-C₃₀); und
(ii) Mono-, Di- oder Triglycerylfettsäureestern (C₄-C₃₀); und
eine druckempfindliche Acrylklebermatrix, die aus einem COOH-Gruppen enthaltenden Copolymer gebildet ist und in der das NSAID enthalten ist.

2. Vorrichtung zur transdermalen Verabreichung nach Anspruch 1, worin das NSAID in einer Menge im Bereich von 4,5 bis 32 Gew.-%, bezogen auf den Gesamtgehalt mit Ausnahme von Kleberlösungsmitteln, enthalten ist.

3. Vorrichtung zur transdermalen Verabreichung nach Anspruch 2, worin das NSAID Diclofenac-diethylammoniumsalz ist.

4. Vorrichtung zur transdermalen Verabreichung nach einem der Ansprüche 1 bis 3, worin die druckempfindliche Klebermatrix Methacrylsäure oder einen Ester davon, zumindest ein anderes copolymerisierbares Monomer und Vernetzer enthält und die druckempfindliche Kleberpolymermatrix in einer Menge im Bereich von 50 bis 97,5 Gew.-% des Gesamtgehalts mit Ausnahme von Kleberlösungsmitteln enthalten ist.

5. Vorrichtung zur transdermalen Verabreichung nach Anspruch 4, worin das druckempfindliche Acrylgemisch ein Copolymer aus 2-Ethylhexylacrylat, Methylacrylat, Acrylsäure und Glycidylmethacrylat ist.

6. Vorrichtung zur transdermalen Verabreichung nach einem der Ansprüche 1 bis 5, worin der Penetrationsverstärker Oleylalkohol ist und in einer Menge im Bereich von 5,5 bis 35 Gew.-%, bezogen auf den Gesamtgehalt mit Ausnahme von Kleberlösungsmitteln, enthalten ist.

7. Vorrichtung zur transdermalen Verabreichung nach einem der Ansprüche 1 bis 5, worin der Penetrationsverstärker Glycerylmonooleat ist und in einer Menge im Bereich von 5,5 bis 35 Gew.-%, bezogen auf den Gesamtgehalt mit Ausnahme von Kleberlösungsmitteln, enthalten ist.

8. Vorrichtung zur transdermalen Verabreichung nach einem der Ansprüche 1 bis 7, worin das Copolymer -OH-Gruppen enthält.

## Revendications

1. Dispositif d'administration transdermique qui contient :
(a) une composition pharmaceutique pour l'administration généralisée d'un NSAID qui est diclofénac ou son sel pharmaceutiquement acceptable ;
(b) une couche d'appui ; et
(c) une couche de décollage ;
où ladite composition pharmaceutique comprend :
au moins un composant qui améliore la perméation percutanée sélectionné parmi :
(i) alcools gras (C₄-C₃₀) ; et
(ii) esters d'acides gras de mono, di ou triglycéryle (C₄-C₃₀) ; et
une matrice adhésive acrylique sensible à la pression formée par un copolymère contenant des groupes -COOH, où est incorporé le NSAID.

2. Dispositif d'administration transdermique selon la revendication 1, où NSAID est présent en une quantité dans la gamme de 4,5 à 32% en poids de la teneur totale à l'exclusion des solvants de l'adhésif.

3. Dispositif d'administration transdermique selon la revendication 2, où NSAID est le sel de diéthylammonium du diclofénac.

4. Dispositif d'administration transdermique selon l'une des revendications 1 à 3, où la matrice adhésive sensible à la pression contient de l'acide méthacrylique ou son ester, au moins un autre monomère copolymérisable et un agent réticulant, et ladite matrice polymérique adhésive sensible à la pression est présente en une quantité dans la gamme de 50 à 97,5% en poids de la teneur totale à l'exclusion des solvants de l'adhésif.

5. Dispositif d'administration transdermique selon la revendication 4, où le mélange acrylique sensible à la pression est un copolymère de 2-éthyl-hexyl-acrylate, acrylate de méthyle, acide acrylique et méthacrylate de glycidyle.

6. Dispositif d'administration transdermique selon l'une quelconque des revendications 1 à 5, où l'agent améliorant la perméation est de l'alcool oléylique, et est présent en une quantité dans la gamme de 5,5 à 35% en poids de la teneur totale à l'exclusion des solvants de l'adhésif.

7. Dispositif d'administration transdermique selon l'une quelconque des revendications 1 à 5, où l'agent améliorant la perméation est le monooléate de glycéryle, et il est présent en une quantité dans la gamme de 5,5 à 35% en poids de la teneur totale à l'exclusion des solvants de l'adhésif.

8. Dispositif d'administration transdermique selon l'une quelconque des revendications 1 à 7, où ledit copolymère contient des groupes -OH.
